# EUROPEAN PATENT APPLICATION

(11) **EP 4 716 436 A1**
(43) Date of publication of application: **25.03.2026**
(21) Application number: 24201408.2
(22) Date of filing: 19.09.2024
(51) Int. Cl.: H10K 50/155, H10K 59/35, H10K 85/30

(54) **OLED DISPLAY COMPRISING CERIUM-DIKETONATE COMPLEXES AND METHOD TO PRODUCE SUCH AN OLED DISPLAY**

(71) Applicant: CREDOXYS GmbH, 01187 Dresden (DE)
(72) Inventor: EYMANN, Léonard, 01187 Dresden (DE); PAPMEYER, Marcus, 01187 Dresden (DE); DOROK, Sascha, 01187 Dresden (DE); STOLZ, Julia, 01187 Dresden (DE)

(57) **Abstract**

The present invention relates to OLED display comprising a plurality of OLED pixels. Further, the present invention relates to a doped semiconductor matrix material and a method to produce such an OLED display. Due to the application of new cerium (IV) complexes as dopants advantageous properties of the doped semiconductor matrix material as well as of the OLED display can be achieved. As an advantage, high evaporation temperatures of the dopants are achieved allowing for mass production and lower lateral conductivity of the doped semiconductor matrix material leads to improved properties of the devices.

## Description

The present invention relates to an OLED display comprising a plurality of OLED pixels. Further, the present invention relates to a doped semiconductor matrix material and a method to produce such an OLED display.

### BACKGROUND OF THE INVENTION

Organic electronics deals with the development, characterization, and application of new materials, both based on small organic molecules and polymers with certain desired electronic properties for the fabrication of electronic components. These include, for example, organic field-effect transistors (OFETs), such as organic thin-film transistors (OTFTs), organic electroluminescent devices, such as organic light-emitting diodes (OLEDs), organic solar cells (OSCs), e.g. exciton solar cells, dye-sensitized solar cells (DSSCs), or perovskite solar cells; electrophotography, e.g., photoconductive materials in organic photoconductors (OPCs); organic optical detectors; organic photoreceptors; light-emitting electrochemical cells (LECs); and organic laser diodes.

It is known that semiconductor matrix materials, in particular organic semiconductor materials, can be strongly influenced in terms of their electrical conductivity by doping. Such semiconductor matrix materials can be built either from compounds with electron donor properties (p-type conductors) or from compounds with electron acceptor properties (n-type conductors). Unlike inorganic semiconductors, organic semiconductors have very low intrinsic charge carrier density. To achieve good semiconductor properties, organic semiconductor matrix materials can be doped, i.e. molecules are introduced into the intrinsic semiconductor matrix material. For n-doping, strong electron donors (n-dopants) are used to transfer an electron to the LUMO (lowest unoccupied molecular orbital) of the semiconductor matrix (n-doping), resulting in a free electron on the matrix (SOMO - singly occupied molecular orbital). For p-doping, strong electron acceptors (p-dopants) are used to remove an electron from the HOMO (highest occupied molecular orbital) of the semiconductor matrix (p-doping), leaving a hole. In other words, for effective p-doping, the LUMO of the dopant must be below the HOMO energy of the matrix. The p-dopant acts as an acceptor for an electron, leaving a moving hole (SOMO) in the matrix.

Known p-dopants for electron donor materials are electron acceptors such as tetracyanoquinone methane (TCNQ), 2,3,5,6-tetrafluorotetracyano-1,4-benzoquinone methane (F4TCNQ), trinaphthylenes (HATNA), metal oxides such as MoOa or WO₃, or radialenes as described in EP 2180029. The acceptor molecules create so-called holes by electron transfer processes in the semiconductor matrix materials, and the conductivity of the semiconductor matrix material (hole transport material) is changed to a greater or lesser extent depending on the number and mobility of the holes.

One type of organic electronic devices are displays consisting of a plurality of OLED-pixels which are arranged on a substrate. The OLED technology is highly suited to be used in the production of lighting modules - as OLEDs are efficient, flexible, lightweight and with excellent lighting properties. OLEDs consist of several layers. In particular, an OLED comprises an anode, a cathode, an emitter material, and charge carrier transport layers such as an electron transport layer and a hole transport layer.

An OLED emitter material emits light in a specific color such as green or red. To realize a display, a classic OLED design (called RGB) uses three emitter materials in RGB subpixels to create a single full-color pixel. As an alternative, red, green, and blue emissions are obtained from white-light OLEDs combined with different color filters. As a further alternative, red and green emissions are obtained from blue OLEDs combined with colour conversion layers, such as quantum dot layers.

In order to gain a high resolution of such a display, the pixels are generally controlled individually. Therefore, it is required to directly access and switch each individual pixel ON or OFF.

It is known that a major problem is the pixel crosstalk, i.e. the unintentional light-up of pixels. As a disadvantage, photons of a second pixel are emitted unintentionally while the neighboring first pixel is switched ON, leading to a reduction of contrast and color gamut. The reason for this problem are common charge transport layers shared by all pixels. When a driving voltage or current is applied to the first pixel, a small current also flows through the floating neighboring pixels. Accordingly, the applied driving current or voltage may cause unintentional light emission from another pixel close to the first pixel.

As a disadvantage, a high conductivity of common charge transport layers leads to high pixel crosstalk. In particular, the high conductivity of p-doped hole transport layers, such as the hole injection layer and the p-doped hole transport layer as comprised in a charge generation layer or pn-junction, are a problem. On the other side, as a low conductivity disadvantageously leads to high voltages required to drive OLED emission, the conductivity must not be too low, but it preferably needs to be in a certain advantageous range above 10⁻⁶ S/cm.

The conductivity of doped charge transport layer is controlled by the dopant concentration. To advantageously reduce the conductivity of the charge transport layer, the dopant concentration can be reduced. However, smaller concentrations are more difficult to control in OLED fabrication. Therefore, to achieve high production yield, it is advantageous to use higher concentrations, e.g. at least 2 vol%, preferably at least 4 vol%. Furthermore, small dopant concentrations can lead to an increase of voltage over time and thus instable device performance. Accordingly, the reduction of the conductivity of a doped layer is difficult without affecting production yield and OLED stability.

This problem of too high conductivities especially applies for hole transport materials (HTMs) with shallow HOMOs, particularly with a HOMO ≥ -5.5 eV when determined using ultraviolet photoelectron spectroscopy or ≥ -5.2 eV when determined using cyclic voltammetry as described herein, especially above the HOMO of BPAPF (9,9-bis[4-(N,N-bis-biphenyl-4-yl-amino)phenyl]-9H-fluorene), when determined under the same conditions. Materials with such shallow HOMOs are for example N-(9,9-Diphenyl-9H-fluoren-2-yl)-N,9-diphenyl-9H-carbazol-3-amine) and BCFA (N-([1,1'-biphenyl]-4-yl)-9,9-dimethyl-N-(4-(9-phenyl-9H-carbazol-3-yl)phenyl)-9H-fluoren-2-amine). The shallow HOMO levels allow for a very efficient doping process using state-of-the art p-dopants resulting in high conductivities that are larger than 1·10⁻⁵ S/cm for doping ratios of only 2 - 4 vol%.

Additionally, the compounds require an evaporation temperature above 120°C to reduce the risk of cross contamination during an evaporation process and to be sufficient for mass production.

WO 2021/048044 A1 and WO 2022/189431 A1 relate to cerium (IV) diketonate complexes as electron acceptors, specifically to be used as p-dopants and electron transport materials in organic electronic components. It has been generally shown that the doping of certain matrix materials using cerium (IV) diketonate complexes is possible, but the compounds disclosed in WO 2021/048044 and WO 2022/189431, respectively, disadvantageously lead to highconductivities that reduce the contrast and the color gamut of the OLED display.

Accordingly, there is an ongoing demand for compounds that have improved properties, in particular with regard to their acceptor strength and resulting conductivities.

Therefore, it is an object of the invention to provide compounds, provide cerium-diketonate complexes, which have improved properties, especially sufficient doping properties for matrix materials with shallow HOMO levels.

### SUMMARY OF THE INVENTION

Surprisingly, it has now been found that specific cerium (IV) diketonate complexes can be applied particularly well as p-dopants in OLED displays.

A first object of the invention relates to an OLED display, in particular an active-matrix OLED **display,** comprising a plurality of OLED pixels, each of the OLED pixels comprising
- An anode,
- a cathode,
- at least a first **emission layer,** and

wherein at least a first OLED pixel of the plurality of pixels and a second OLED pixel of the plurality of pixels comprise a cathode that forms a **common cathode,**
wherein at least the first OLED pixel of the plurality of pixels and the second OLED pixel of the plurality of pixels comprise a first p-type semiconductor layer that forms at least a first **common p-type semiconductor layer,**
wherein the first common p-type semiconductor layer comprises a compound of the general formula (I)

   Ce⁴⁺(L_{A}L_{B}L_{C}L_{D})⁴⁻ (I),
wherein
L_{A}; L_{B}; L_{C}; and L_{D} are independently from each other selected from a bidentate ligand having the general formula (II) 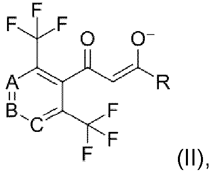
wherein A and C are independently selected from N (nitrogen), C (carbon)-H, C-D, CAr, C-OCF₃, C-SCF₃, SO₂CF₃, CN, F and CF₃;
wherein B is selected from N, C-D, C-Ar, C-H;
wherein R represents CF₃ or C₂F₅ or C₃F₇.

The OLED may further comprise a driving circuit configured to separately driving the pixels of the plurality of OLED pixels.

A further object of the invention relates to a doped semiconductor matrix material comprising at least one electron donor and at least one compound of the formula (I) according to the invention and as defined herein, wherein the electron donor is preferably a triaryl amine or carbazole based compound, comprising at least one or more nitrogen atoms exhaustively substituted by aromatic moieties.

A further object relates to a compound of the general formula (I)

Ce⁴⁺(L_{A}L_{B}L_{C}L_{D})⁴⁻ (I),

wherein
L_{A}; L_{B}; L_{C}; and L_{D} are independently from each other selected from a bidentate ligand having the general formula (II) 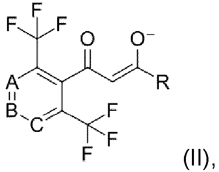
wherein A and C are independently selected from N (nitrogen), C (carbon)-H, C-D, C-Ar, C-OCF₃, C-SCF₃, SO₂CF₃, CN, F and CF₃;
wherein B is selected from N, C-D, C-Ar, C-H;
wherein R represents CF₃ or C₂F₅ or C₃F₇.

A further object of the invention is a method to produce an OLED display according to the invention, wherein the first common p-type semiconductor layer comprises a compound of general formula (I), comprising at least one of the following steps:
(a) Simultaneous evaporation of the semiconductor matrix material and the compound of formula (I),
(b) Evaporation of a pre-mixed material containing the semiconductor matrix material and at least one compound of general formula (I) from one single source,
(c) Sequential deposition of the semiconductor matrix material and at least one compound of general formula (I),
(d) Doping of a semiconductor matrix material layer by a solution of at least one compound of general formula (I) followed by evaporation of the solvent by thermal treatment,
(e) Surface doping of a layer of a semiconductor matrix material by a layer of at least one compound of general formula (I) applied to one or both surfaces of the layer of a semiconductor matrix material,
(f) Preparing a solution of a semiconductor matrix material and at least one compound of general formula (I) and forming a film from the solution.

### DESCRIPTION OF THE INVENTION

The invention has the following advantages:
- The cerium (IV) complexes applied for the invention have only low manufacturing costs.
- The cerium (IV) complexes according to the invention are advantageously suitable as electron acceptors for use as p-dopants in OLED displays.
- The cerium (IV) complexes according to the invention lead to an optimized conductivity of the doped semiconductor matrix material, particularly in semiconductor matrix materials with a shallow HOMO level, particularly ≥ -5.5 eV when determined using ultraviolet photoelectron spectroscopy or ≥ -5.2 eV when determined using cyclic voltammetry as described herein, especially as shallow or above the HOMO level of BPAPF, in particular the compounds of general formular (I) are advantageously suitable for a doping of semiconductor matrix materials with a HOMO between the HOMO of BPAPF and ZnPc (Zinc Phtalocyanine), while they are suitable for the production of organic and hybrid opto-electronic devices, in particular for OLEDs and OLED displays, both by solvent processing and due to their high evaporation temperature also by vacuum processing.
- The cerium (IV) complexes applied for the invention show improved thermostability of the doped layers compared to the prior art. Therefore, the OLED displays show an improved thermostability.
- In addition, the cerium (IV) complexes according to the invention are characterized by an advantageous lower doping efficiency, i.e. an advantageous smaller number of free charges within the matrix material gained for an identical number of doping molecules, i.e., a number of doping molecules that still allows for reproducible manufacturing processes.
- The cerium (IV) complexes according to the invention exhibit only a low absorption of the doped layer. Thus, parasitic absorption and emission can be reduced or even prevented.
- The cerium (IV) complexes according to the invention have typically less than 10% source residue while being evaporated using high evaporation rates.
- The cerium (IV) complexes according to the invention are stable for several days at high temperatures as used during evaporation in mass production.
- Due to the application of new cerium (IV) complexes as dopants advantageous properties of the doped semiconductor matrix material as well as of the OLED display can be achieved. As an advantage, high evaporation temperatures of the dopants are achieved allowing for mass production and lower lateral conductivity of the doped semiconductor matrix material leads to improved properties of the devices.

Surprisingly, it has been found that cerium(IV) complexes having diketonate ligands containing at least one unfunctionalized or functionalized bisorthotrifluoromethyl-substituted acyl group
are particular suitable for the doping in a certain desirable range. Surprisingly, the doping efficiency is not too high, but in a desirable range for display applications. Accordingly, displays comprising cerium compounds according to formula (I) show advantageous properties such as:
- Advantageous evaporation temperature and therefore suitable for mass production
- Low pixel crosstalk and therefore suitable for the production of OLED displays with high contrast and color gamut

These advantages particularly apply in combination with a matrix material having a HOMO shallower or equal to the HOMO of BPAPF if measured with the same method, in particular a matrix material with a HOMO shallower than BPAPF and deeper than ZnPc (zinc phthalocyanine), for example BCFN (N-([1,1'-biphenyl]-4-yl)-9,9-dimethyl-N-(4-(9-phenyl-9H-carbazol-3-yl)phenyl)-9H-fluoren-2-amine) and/or EHT003 (N-(9,9-Diphenyl-9H-fluoren-2-yl)-N,9-diphenyl-9H-carbazol-3-amine).

In the production of OLED displays, typically HTMs with shallow HOMO are used for common hole transport layers. These HTMs are readily available from different suppliers and ensure low operating voltages by good energy level alignment with indium tin oxide, the most common anode material. Therefore, it is a critical aspect to gain the possibility of doping HTMs with shallow HOMOs, while generating conductivities that are in a suitable range, in particular between 1·10⁻⁶ S/cm and 3·10⁻⁵ S/cm, preferably between 2·10⁻⁶ S/cm and 2·10⁻⁵ S/cm, more preferably lower than 5·10⁻⁶ S/cm to obtain both low operating voltage of the OLED and low lateral currents leading to pixel crosstalk.^

The displays according to the invention comprise compounds that show advantageous evaporation temperatures in combination with desirable conductivities if combined with matrix materials whose HOMO is shallower or equal to the HOMO of BPAPF.

Surprisingly, it has been found that the electron withdrawing effect of the CF₃ groups is unexpectantly small, if they are positioned on 2 and 6. Even if combined with CF₃ groups on position 3 and 5, i.e. here referred to as A and C, the electron withdrawing effect of the compounds is comparably small, in particular in an advantageous range.

The terms cerium compound and cerium complex are used synonymously and are defined by formula (I). The ligands in the absence of the metal atom (cerium atom), are defined by formula (II).

In the context of the present specification the term "compound of the general formula (I)" means a compound that corresponds to the formula (I) according to the invention as defined herein. An electronic component which comprises a compound of the general formula (I) may comprise each compound which is included in one of the preferred embodiments of the compounds as described herein. This also applies to the term "compound of the formula (I)".

The term reduction products of a compound of formula (I) are anion complexes, especially Ce(lll) anion complexes, where the corresponding counterion is formed from the hole-transport material (HTL) of the matrix.

The term charge transfer complexes of a compound of formula (I) means its ionic pairings with the radical cations of the semiconductor matrix material (hole transport material, HTM).

In the context of the invention, a bidentate ligand (also called didentate) is a ligand, which binds with two atoms to the metal atom (cerium atom).

In the context of the invention, formula (II) represents an exemplary mesomeric frontier structure to which the ligands L are not restricted. Other mesomeric structures are possible.

In the context of the invention, a bidentate ligand (also called a bidentate) is a ligand that binds to the metal atom (cerium atom) with two atoms.

In the context of the invention, the prefix Cₙ-Cₘ indicates the number of carbon atoms that a molecule or residue designated thereby may contain.

In the context of the invention, the expression C₁-C₆-alkyl refers to unbranched or branched saturated hydrocarbon groups having 1 to 6 carbon atoms. C₁-C₆-alkyl are e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl. C₁-C₄-alkyl refers, e.g. to methyl, ethyl, propyl, 1-methylethyl, butyl, 1-methylpropyl, 2-methylpropyl or 1,1-dimethylethyl.

In the context of the invention the expression C₁-C₆-alkoxy refers to an unbranched or branched saturated C₁-C₆-alkyl group as defined above, which is bound via an oxygen atom. Alkoxy radicals with 1 to 4 carbon atoms are preferred, particularly preferred are 1 or 2 carbon atoms. C₁-C₂-alkoxy is methoxy or ethoxy. C₁-C₄-alkoxy is e.g. methoxy, ethoxy, n-propoxy, 1-methylethoxy (isopropoxy), butoxy, 1-methylpropoxy (sec-butoxy), 2-methylpropoxy (isobutoxy) or 1,1-dimethylethoxy (tert-butoxy). C₁-C₆-alkoxy comprises the meanings given for C₁-C₄-alkoxy and additionally e.g. pentoxy, 1 methylbutoxy, 2-methylbutoxy, 3-methylbutoxy, 1,1-dimethylpropoxy, 1,2-dimethylpropoxy, 2,2-dimethylpropoxy, 1-ethylpropoxy, hexyloxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy and 3,3-dimethylbutoxy.

In the context of the invention the expression C₁-C₆-alkylsulfanyl refers to an unbranched or branched saturated C₁-C₆-alkyl group as defined above, which is bound via a sulfur atom. Alkylsulfanyl radicals with 1 to 4 carbon atoms are preferred, particularly preferred are 1 or 2 carbon atoms. C₁-C₂-alkylsulfanyl is methylsulfanyl or ethylsulfanyl. C₁-C₄-alkylsulfanyl is e.g. methylsulfanyl, ethylsulfanyl, n-propylsulfanyl, 1-methylethylsulfanyl (isopropylsulfanyl), butylsulfanyl, 1-methylpropylsulfanyl (sec-butylsulfanyl), 2-methylpropylsulfanyl (isobutylsulfanyl) or 1,1-dimethylethylsulfanyl (tert-butylsulfanyl). C₁-C₆-alkylthio comprises the meanings given for C₁-C₄-alkylsulfanyl and additionally also, e.g., pentylsulfanyl, 1-methylbutylsulfanyl, 2-methylbutylsulfanyl, 3-methylbutylsulfanyl, 1,1-dimethylpropylsulfanyl, 1,2-dimethylpropylsulfanyl, 2,2-dimethylpropylsulfanyl, 1-ethylpropylsulfanyl, hexylsulfanyl, 1-methylpentylsulfanyl, 2-methylpentylsulfanyl, 3-methylpentylsulfanyl, 4-methylpentylsulfanyl, 1,1-dimethylbutylsulfanyl, 1,2-dimethylbutylsulfanyl, 1,3-dimethylbutylsulfanyl, 2,2-dimethylbutylsulfanyl, 2,3-dimethylbutylsulfanyl, 3,3-dimethylbutylsulfanyl, 1-ethylbutylsulfanyl, 2-ethylbutylsulfanyl, 1,1,2-trimethylpropylsulfanyl, 1,2,2-trimethylpropylsulfanyl, 1-ethyl-1-methylpropylsulfanyl or 1-ethyl-2-methylpropylsulfanyl.

In the context of the invention the expressions haloalkyl, haloalkoxy and haloalkylsulfanyl refer to partially or fully halogenated alkyl, alkoxy or alkylsulfanyl. In other words, one or more hydrogen atoms, for example 1, 2, 3, 4 or 5 hydrogen atoms bonded to one or more carbon atoms of alkyl, alkoxy or alkylsulfanyl are replaced by a halogen atom, in particular by fluorine or chlorine.

The expression "halogen" denotes in each case fluorine, chlorine, bromine or iodine.

The expression "CN" denotes the cyano group (-C=N).

The expression "aryl" comprises in the context of the invention mono- or polynuclear aromatic hydrocarbon radicals with usually 6 to 14, especially preferably 6 to 10 carbon atoms. Examples of aryl are especially phenyl, naphthyl, indenyl, fluorenyl, anthracenyl, phenanthrenyl, naphthacenyl, chrysenyl, pyrenyl, etc. and especially phenyl or naphthyl.

The expression "hetaryl" comprises in the context of the invention mononuclear aromatic hydrocarbon radicals with 4 to 5 carbon atoms, wherein 1, 2 or 3 carbon atoms have been replaced by 1, 2 or 3 nitrogen atoms as ring members. The hetaryl group may be attached to the remainder of the molecule via a ring carbon or via a ring nitrogen. Examples of 5- or 6-membered aromatic heterocyclic rings (also called heteroaromatic rings or hetaryl) are 2-pyrrolyl, 3-pyrrolyl, 3-pyrazolyl, 4-pyrazolyl, 5-pyrazolyl, 2-imidazolyl, 4-imidazolyl, 1,3,4-triazol-2-yl, 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

In the context of the invention the term "C₅-C₇-cycloalkyl" as used herein refers to a monocyclic membered saturated cycloaliphatic radicals, e.g. cyclopentyl, cyclohex- yl, cycloheptyl. The term C₅-C₇-halocycloalkyl as used herein, which is also expressed as "cycloalkyl which is partially or fully halogenated", refers C₅-C₇-cycloalkyl as mentioned above, in which some or all of the hydrogen atoms are replaced by halogen atoms as mentioned above, in par-ticular fluorine.

In the context of the present specification the term "electron donor" means a compound with electron donor properties which is an element or a chemical compound which transfers an electron to another compound including the possible formation of a charge transfer complex. In the context of this specification an electron donor is a semiconductor material, in particular a semiconductor matrix material, which may be used as a hole transport material and therefore may form a hole transport layer, particularly if combined with a dopant which is a compound of the general formula (I).

In the context of the present specification, "triaryl amine based compound" means a compound in which at least one nitrogen atom is bonded to three aryl groups via three covalent bonds, i.e. there are three covalent bonds, each of which bonds the nitrogen atom to at least one aryl group.

In the context of the present specification, "carbazole based compound" means a compound in which one nitrogen atom of the carbazole unit is bonded to one aryl group via a covalent bonds.

In the context of the invention, HOMO or HOMO level mean the HOMO energy of the respective molecule.

In the context of the present specification the term "hole transport material" means a semiconductor matrix material. In combination with a compound suitable for an application as p-dopant, the hole transport material and the compound suitable for an application as p-dopant may form a hole transport layer. In the context of the invention, a hole transport layer may comprise a hole transport material or a hole transport layer may comprise a hole transport material and a p-dopant; furthermore, a hole transport layer may consist of a hole transport material only. In general, a semiconductor matrix material combined with a dopant or with a compound suitable as a dopant is a doped semiconductor.

### Cerium-Compounds of formula (I)

Suitable cerium(IV) compounds in the sense of the invention are the compounds of the general formula (I)

Ce⁴⁺(L_{A}L_{B}L_{C}L_{D})⁴⁻ (I),

wherein
L_{A}; L_{B}; Lc; and L_{D} are defined as above and below, encompasses compounds, wherein
- all four ligands L_{A}, L_{B}, Lc, and L_{D} have the same meaning, or
- three of the four ligands have the same meaning, or
- two of the four ligands have the same meaning, or
- all four ligands L_{A}, L_{B}, Lc, and L_{D} have different meanings.

Preferred are compounds of formula (I) wherein L_{A}, L_{B}, Lc, and L_{D} have the same meaning.

In the cerium compounds of formula (I) L_{A}, L_{B}, L_{C}, and L_{D} are independently from each other selected from bidentate ligands having the general formula (II). In the following preferred embodiments of the compounds (I) are directly defined by preferred embodiments of their bidentate ligands (II).

In one embodiment, L_{A}; L_{B}; L_{C}; and L_{D} are similar.

In one embodiment, A and C are independently selected from N (nitrogen), C (carbon)-H, C-D, C-Ar, C-OCF₃, C-SCF₃, SO₂CF₃, CN, and F. Due to the reduction of possible CF₃ groups the electron withdrawing effect is limited to even smaller values. Accordingly, a pixel cross talking can be prevented within the OLED display.

In an optional embodiment L_{A}; L_{B}; L_{C}; and L_{D} are independently from each other selected from

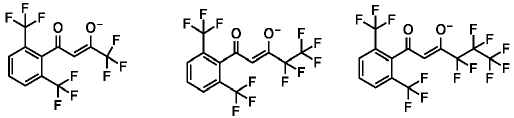

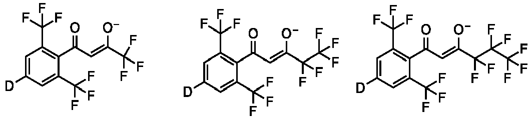

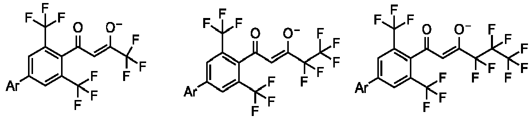

### Display

Preferred are OLED displays, in particular an active matrix OLED display, comprising a plurality of OLED pixels, each of the OLED pixels comprising
- an anode,
- a cathode,
- at least a first emission layer, and

wherein at least a first OLED pixel of the plurality of pixels and a second OLED pixel of the plurality of pixels comprise a cathode that forms a common cathode,
wherein at least the first OLED pixel of the plurality of pixels and the second OLED pixel of the plurality of pixels comprise a first p-type semiconductor layer that forms at least a first common p-type semiconductor layer,
wherein the first common p-type semiconductor layer comprises a compound of the general formula (I)

   Ce⁴⁺(L_{A}L_{B}L_{C}L_{D})⁴⁻ (I),
wherein
L_{A}; L_{B}; Lc; and L_{D} are defined as above and below, encompasses compounds, wherein
   - all four ligands L_{A}, L_{B}, Lc, and L_{D} have the same meaning, or
   - three of the four ligands have the same meaning, or
   - two of the four ligands have the same meaning, or
   - all four ligands L_{A}, L_{B}, Lc, and L_{D} have different meanings.

In the cerium compounds of formula (I) L_{A}, L_{B}, L_{C}, and L_{D}are independently from each other selected from bidentate ligands having the general formula (II). In the following preferred embodiments of the compounds (I) are directly defined by preferred embodiments of their bidentate ligands (II).

In the context of the present specification the term "charge generation layer" is related to a p-type semiconductor layer that is positioned in vicinity of an n-type semiconductor layer between two emission layers and is built to enable the charge carriers to be converted in such a way that both emission layers can be driven. The p-type and the n-type semiconductor layers of the charge generation layer may be directly adjacent to each other or separated by an interlayer.

In the context of the present specification the term "common layer" refers to a layer that is shared between at least two subpixels or pixels.

Preferably, the electronic component also comprises a substrate (a metal, glass, or plastic substrate).

In a preferred embodiment, the first subpixel is built to emit light of a first color, e.g. red, and the second subpixel is built to emit light of a second color, e.g. blue or green. The first common p-type semiconductor layer is a common layer which is shared between the first and the second subpixel. Preferably, the first common layer is shared between at least 2 full-color pixels, more preferably between at least 100 full-color pixels.

Preferably at least 100 OLED pixels of the plurality of pixels comprise a cathode that forms a common cathode and at least 100 OLED pixels of the plurality of pixels comprise a first p-type semiconductor layer that form the first common p-type semiconductor layer. As an option all pixels comprise first p-type semiconductor layers that form a first common p-type semiconductor layer.

The first emission layer can also form a common emission layer. Preferably, the first OLED pixel and the second OLED pixel, preferably at least 10 pixels, more preferably at least 100 pixels, comprise first emission layers that form at least a first **common emission layer.** The OLED display according to one of the claims 1 to 9, wherein the first OLED pixel and the second OLED pixel, comprise first emission layers that form at least a first common emission layer.

The OLED display according to one of the claims 1 to 10, wherein the first OLED pixel and the second OLED pixel comprise a stack of organic layers that form a stack of common organic layers.

In a preferred embodiment, the first OLED pixel and the second OLED pixel comprise a stack of organic layers that form a stack of common organic layers.

In one embodiment, each of the layers of a pixel may form a common layer.

In one embodiment, L_{A}; L_{B}; L_{C}; and L_{D} are similar.

In one embodiment, A and C are independently selected from N (nitrogen), C (carbon)-H, C-D, C-Ar, C-OCF₃, C-SCF₃, SO₂CF₃, CN, and F. Due to the reduction of possible CF₃ groups the electron withdrawing effect is limited to even smaller values. Accordingly, a pixel cross talking can be prevented within the OLED display.

In an optional embodiment L_{A}; L_{B}; L_{C}; and L_{D} are independently from each other selected from

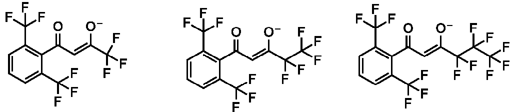

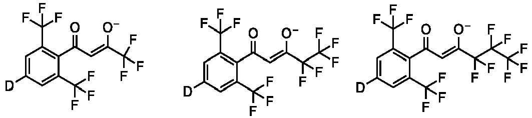

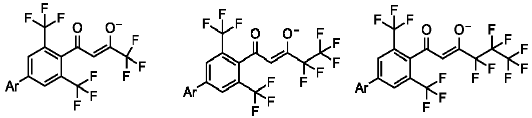

In an optional embodiment L_{A}; L_{B}; Lc; and L_{D} are similar and selected from

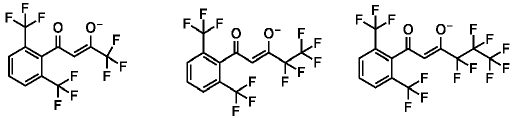

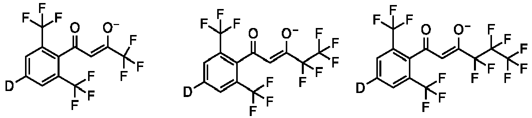

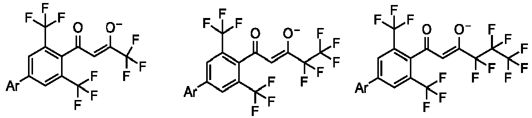

In a preferred embodiment, the first common p-type semiconductor layer is a hole transport layer and/or a hole injection layer and/or a charge generation layer.

In one embodiment the first common p-type semiconductor layer is positioned between the anode and the first emission layer, wherein the p-type semiconductor layer is for example a hole transport layer and/or a hole injection layer.

A hole injection layer is generally a layer that facilitates hole injection from the anode into the semiconductor matrix material. The hole injection layer can be placed directly adjacent to the anode. The anode may or may not be composed of several sublayers. The hole injection layer may be purely composed of a compound of formula (I). Preferably, the hole injection layer is composed of a semiconductor matrix material and one or more compound(s) of formula (I).

Preferably, the hole transport layer is composed of a semiconductor matrix material and one or more compound(s) of formula (I). Specifically, the hole transport layer may be located between a hole injection layer and an emitting layer, where it transports holes from the anode to the emitting layer. Between the hole transport layer and the emitting layer may be other layers, such as electron blocking layers, exciton blocking layers, and prime layers. In another embodiment, preferably a tandem- or triple-stack OLED, the hole transport layer transports holes from a charge generation layer to an emitting layer. Between the hole transport layer and the emitting layer may be other layers, such as electron blocking layers, exciton blocking layers, and prime layers.

In one embodiment the first OLED pixel and the second OLED pixel of the plurality of the OLED pixels comprise at least a second emission layer and the first common p-type semiconductor layer is a first p-type charge generation layer. Thereby the first p-type charge generation layer is part of a first charge generation layer which is arranged between the first emission layer and the second emission layer.

This kind of OLED display may comprise further common layers, in particular a common p-type semiconductor layer between the anode and the first emission layer.

In certain embodiments, the hole transport layer has a thickness which is smaller than or equal to 200 nm.

Preferably, the hole injection layer has a thickness in a range from 2 to 20 nm, more preferably, between 5 nm and 10 nm.

In a preferred embodiment of the OLED display the hole injection layer is positioned adjacent to one of the electrodes, especially the anode.

In one embodiment the first common p-type semiconductor layer comprises a semiconductor **matrix material.** Preferably, the HOMO level of the semiconductor matrix material, especially the hole transport material, is equal to or higher than the HOMO of BPAPF if measured with the same method.

As an option the HOMO level of the matrix material is ≥-5.2 eV when determined using cyclic voltammetry.

In one embodiment, the HOMO level of the matrix material is ≤ -4,8 eV when determined using cyclic voltammetry.

The first common p-type semiconductor layer may consist of at least **two sublayers,** wherein the sublayer arranged next to the anode comprises a compound according to formula (I) and the adjacent sublayer comprises a matrix material. The first common p-type semiconductor layer may even comprise additional sublayers, for example blocking layers.

The first common p-type semiconductor layer may comprise a doped matrix material, wherein the matrix material is mixed with a compound according to formula (I).

In one embodiment the matrix material and the compound according to formula (I) arranged in a **mixed layer,** for example the compound according to formula (I) is doped into the matrix material.

In one embodiment the **lateral conductivity** of the first common p-type semiconductor layer is smaller than 3 E-5 S/cm, preferably smaller than 2 E-5 S/cm, wherein the lateral conductivity is measured by a transmission line method at room temperature. Preferably, a plurality of the OLED pixels comprises at least a **second emission layer** and the first common p-type semiconductor layer is a first p-type charge generation layer which is a part of a first charge generation layer, wherein the first charge generation layer is arranged between the first emission layer and the second emission layer. Such an arrangement can be called **tandem stack.**

This kind of OLED display may comprise further common layers, in particular a common p-type semiconductor layer between the anode and the first emission layer.

In a preferred embodiment of the OLED display, in particular in form of a tandem or triple or quadruple-stack OLED display, the first common p-type semiconductor layer which comprises the compound of formula (I) is part of a pn-junction, particularly in form of a charge-generation layer. The pn-junction may connect for example two light emitting units and may be composed of a p-type charge generation layer, an n-type charge generation layer, and optionally one or more interlayers. Preferably, the layer comprising a compound of formula (I) is the p-type charge generation layer. Preferably, this p-type charge generation layer is composed of a semiconductor matrix material and one or more compound(s) of formula (I).

A compound of formula (I) may also be used in an interlayer.

Preferably, the charge generation layer may comprise a first common p-type semiconductor layer comprising a compound according to formula (I). For clarity, the charge generation layer is a common layer of at least the first and the second OLED pixel, preferably of at least 10 pixels.

In one embodiment, the OLED display comprises a second common p-type semiconductor layer which comprises a compound of formula (I) according to the invention. This second common p-type semiconductor layer may be a hole transport layer and/or a hole injection layer and/or a p-type charge generation layer in a tandem OLED display or in a triple OLED display or in a quadruple OLED display. The p-type charge generation layer is part of a charge generation layer.

In one embodiment the second common p-type semiconductor layer may comprise any compound of formula (I) according to the invention.

The second common p-type semiconductor layer may comprise the compound of formula (I) which is also present in the first common p-type semiconductor layer.

The second common p-type semiconductor layer is a common layer which is shared between the first and the second subpixel. Preferably, the second common layer is shared between at least 2 full-color pixels, more preferably between at least 100 full-color pixels.

In one embodiment the second common p-type semiconductor layer is a p-type charge generation layer or a hole injection layer or a hole transport layer in a tandem OLED or in a triple OLED or in a quadruple OLED.

In one embodiment the OLED display comprises a third common p-type semiconductor layer which comprises a compound of formula (I) according to the invention.

The third common p-type semiconductor layer can be a p-type charge generation layer or a hole injection layer or a hole transport layer in a tandem OLED or in a triple OLED or in a quadruple OLED.

In one embodiment a plurality of the OLED pixels, at least the first and the second OLED pixel, comprises a second emission layer, a third emission layer and at least a first charge generation layer and a second charge generation layer. The first charge generation layer is arranged between the first emission layer and the second emission layer and the second charge generation layer is arranged between the second and the third emission layer. This type of stack can be referred to as triple stack. The second charge generation layer may comprise a second p-type charge generation layer. The second p-type charge generation layer may be a third common p-type semiconductor layer comprising a compound of formula (I).

In one embodiment, the OLED display is in the form of a quadruple stack display.

The OLED display may also comprise other layers, for example, one or more each of hole blocking layers, electron transport layers, electron injection layers, electron blocking layers, exciton blocking layers, charge conversion contacts and/or charge generation layers, both also referred to as pn-junctions. Not each of these layers must necessarily be present.

In one embodiment, the OLED display may comprise a first common p-type semiconductor layer comprising a compound of formula (I) and it may also comprise a second p-dopant which differs from compounds of formula (I), in particular a p-dopant such as the class of 3-radialenes, preferrably α,α',α"-1,2,3-Cyclopropanetriylidenetris[4-cyano-2,3,5,6-tetrafluorobenzeneacetonitrile], from the class of dicyanochinodimethane-derivatives, preferrably 2,3,5,6-Tetrafluor-7,7,8,8-tetracyanochindimethan, 2,2'-(1,3,4,5,7,8-Hexafluoro-2,6-naphthalenediylidene)bis[propanedinitrile], 2,2'-[4,8-Bis[2,4-bis(trifluoromethyl)phenyl]benzo[1,2-d:4,5-d']bisoxazole-2,6-diylidene]bis[propanedinitrile], from the class of metal complexes, in particular cerium(IV) diketonates and derivatives or from the class of benzobisoxazole and derivatives such as 2,2'-[4,8-Bis[2,4-bis(trifluoromethyl)phenyl]benzo[1,2-d:4,5-d']bisoxazole-2,6-diylidene]bis[propanedinitrile].

The second p-dopant may be present in the same or a different layer or sub-layer than the compound of formula (I). Accordingly, the OLED display may comprise a first common p-type semiconductor layer comprising a compound of formula (I) and it may also comprise a second common p-type semiconductor layer comprising a p-dopant such as mentioned above.

### Semiconductor matrix materials

Preferred is an OLED display, wherein the first common p-type semiconductor layer comprises a matrix material, i.e. a semiconductor matrix material.

The compounds of the formula (I) according to the invention, the charge transfer complexes thereof, and the reduction products thereof can be used as dopants in semiconductor matrix materials, in particular as p-dopants in hole transport layers. The doped semiconductor matrix material preferably comprises at least one electron donor and at least one compound of formula (I), wherein the electron donor is preferably a triaryl amine based compound, comprising at least one or more nitrogen atoms exhaustively substituted by aromatic moieties.

Possible electron donors are diaminoterphenyls, diaminotrimethylphenylindanes, and Di-, tri- and tetraphenylindane amine derivatives.

Suitable diaminoterphenyls are described in DE 102012007795.

Diaminotrimethylphenylindanes are described in WO 2018/206769.

Di-, tri- and tetraphenylindane amine derivatives are described in WO2020094847.

The electron donors can be selected from triarylamines. Especially the electron donors can be selected from 4,4',4"-tris(N-(2-naphthyl)-N-phenyl-amino)triphenylamine (2-TNATA), 4,4',4"-tris(N-3-methylphenyl-N-phenyl-amino)triphenylamine (m-MTDATA), N,N,N',N'-tetrakis(4-methoxy-phenyl)benzidine (MeO-TPD), (2,2',7,7'-tetrakis-(N,N-diphenylamino)-9,9'-spirobifluorene (spiro-TTB), N,N'-bis(naphthalene-1-yl)-N,N'-bis(phenyl)-benzidine, N,N'-bis(naphthalene-1-yl)-N,N'-bis(phenyl)-9,9-spiro-bifluorene, 9,9-bis[4-(N,N-bis-biphenyl-4-yl-amino)phenyl]-9H-fluorene, 2,2'-bis[N,N-bis(biphenyl-4-yl)amino]-9,9-spiro-bifluorene, N,N'-((9H-fluorene-9,9-diyl)bis(4,1-phenylene))bis(N-([1,1'-biphenyl]-4-yl)-[1,1'-biphenyl]-4-amine) (BPAPF), N,N'-bis(phenanthrene-9-yl)-N,N'-bis(phenyl)-benzidine, 1,3,5-tris{4-[bis(9,9-dimethyl-fluorene-2-yl)amino]phenyl}benzene, tri(terphenyl-4-yl)amine, N-(4-(6-((9,9-dimethyl-9H-fluorene-2-yl)(6-methoxy-[1,1'-biphenyl]-3-yl)amino)-1,3,3-trimethyl-2,3-dihydro-1H-indene-1-yl)phenyl)-N-(6-methoxy-[1,1'-biphenyl]-3-yl)-9,9-dimethyl-9H-fluorene-2-amine, N-([1,1'-biphenyl]-4-yl)-N-(4-(6-([1,1'-biphenyl]-4-yl(9,9-dimethyl-9H-fluorene-2-yl)amino)-1,3,3-trimethyl-2,3-dihydro-1H-indene-1-yl)phenyl)-9,9-dimethyl-9H-fluorene-2-amine, N,N-di([1,1'-biphenyl]-4-yl)-3-(4-(di([1,1'-biphenyl]-4-yl)amino)phenyl)-1,1,3-trimethyl-2,3-dihydro-1H-indene-5-amine, N-(4-(6-(bis(9,9-dimethyl-9H-fluorene-2-yl)amino)-1,3,3-trimethyl-2,3-dihydro-1H-inden-1-yl)phenyl)-N-(9,9-dimethyl-9H-fluorene-2-yl)-9,9-dimethyl-9H-fluorene-2-amine, N-(4-(6-(9,9'-spirobi[fluorene]-2-yl(9,9-dimethyl-9H-fluorene-2-yl)amino)-1,3,3-trimethyl-2,3-dihydro-1H-inden-1-yl)phenyl)-N-(9,9-dimethyl-9H-fluoren-2-yl)-9,9'-spirobi[fluorene]-2-amine, N-(4-(6-(dibenzo[b,d]furane-2-yl(9,9-dimethyl-9H-fluorene-2-yl)amino)-1,3,3-trimethyl-2,3-dihydro-1H-indene-1-yl)phenyl)-N-(9,9-dimethyl-9H-fluorene-2-yl)dibenzo[b,d]furan-2-amine, 9-(4-(6-(9H-carbazol-9-yl)-1,3,3-trimethyl-2,3-dihydro-1H-inden-1-yl)phenyl)-9H-carbazole, N-([1,1'-biphenyl]-4-yl)-3-(4-([1,1'-biphenyl]-4-yl(4-methoxyphenyl)amino)phenyl)-N-(4-methoxyphenyl)-1,1,3-trimethyl-2,3-dihydro-1H-inden-5-amine, 3-(4-(bis(6-methoxy-[1,1'-biphenyl]-3-yl)amino)phenyl)-N,N-bis(6-methoxy-[1,1'-biphenyl]-3-yl)-1,1,3-trimethyl-2,3-dihydro-1H-indene-5-amine, N1-([1,1'-biphenyl]-4-yl)-N 1-(4-(6-([1,1'-biphenyl]-4-yl(4-(diphenylamino)phenyl)amino)-1,3,3-trimethyl-2,3-dihydro-1H-inden-1-yl)phenyl)-N4,N4-diphenylbenzene-1,4-diamine, N,N-di([1,1'-biphenyl]-4-yl)-4'-(6-(4-(di([1,1'-biphenyl]-4-yl)amino)phenyl)-1,3,3-trimethyl-2,3-dihydro-1H-indene-1-yl)-[1,1'-biphenyl]-4-amine, N-(4-(5-(bis(9,9-dimethyl-9H-fluorene-2-yl)amino)-1,3,3-trimethyl-2,3-dihydro-1H-indene-1-yl)phenyl)-N-(9,9-dimethyl-9H-fluorene-2-yl)-9,9-dimethyl-9H-fluorene-2-amine, N-(4-(6-(bis(9,9-dimethyl-9H-fluorene-2-yl)amino)-1,3,3-trimethyl-2,3-dihydro-1H-indene-1-yl)phenyl)-N-(9,9-dimethyl-9H-fluorene-2-yl)-9,9-dimethyl-9H-fluorene-2-amine, N,N'-bis(9,9-dimethyl-fluorene-2-yl)-N,N'-diphenyl-benzidine (BF-DPB), N, N'-((9H-fluorene-9,9-diyl)bis(4,1-phenylene))bis(N-([1,1'-biphenyl]-4-yl)-[1,1'-biphenyl]-4-amine) (BPAPF), N4,N4,N4',N4'-tetrakis(9,9-dimethyl-9H-fluorene-2-yl)-[1,1'-biphenyl]-4,4'-diamine (TDMFB), N-([1,1'-biphenyl]-2-yl)-N-(9,9-dimethyl-9H-fluorene-2-yl)-9,9'-spirobi[fluorene]-2-amine, (2,7-bis[N,N-bis(4-methoxyphenyl)amino]-9,9-spirobi[9*H-*fluorene] (spiro-MeO-TPD), a mixture of N-(4-(5-(bis(9,9-dimethyl-9H-fluorene-2-yl)amino)-1,3,3-trimethyl-2,3-dihydro-1H-inden-1-yl)phenyl)-N-(9,9-dimethyl-9H-fluorene-2-yl)-9,9-dimethyl-9H-fluorene-2-amine and N-(4-(6-(bis(9,9-dimethyl-9H-fluorene-2-yl)amino)-1,3,3-trimethyl-2,3-dihydro-1H-inden-1-yl)phenyl)-N-(9,9-dimethyl-9H-fluorene-2-yl)-9,9-dimethyl-9H-fluorene-2-amine, N-([1,1'-biphenyl]-4-yl)-9,9-dimethyl-N-(4-(9-phenyl-9H-carbazol-3-yl)phenyl)-9H-fluorene-2-amine, CAS [866119-24-6], N-(9,9-Diphenyl-9H-fluoren-2-yl)-N,9-diphenyl-9H-carbazol-3-amine, CAS [1069137-74-1], N,N-Di([1,1'-biphenyl]-4-yl)-4'-(9H-carbazol-9-yl)-[1,1'-biphenyl]-4-amine , CAS [1242056-42-3], N-([1,1'-Biphenyl]-4-yl)-9,9-dimethyl-N-(4-(9-phenyl-9H-carbazol-3-yl)phenyl)-9H-fluoren-2-amine, CAS [81364603-07-5], N-([1,1'-Biphenyl]-2-yl)-N-(9,9-dimethyl-9H-fluoren-2-yl)-9,9'-spirobi[fluoren]-2-amine and mixtures thereof.

Another aspect of the invention is a doped semiconductor layer comprising at least one electron donor, which can also be referred to as semiconductor matrix material and at least one doping agent, wherein the doping agent is one or more compounds of formula (I), and/or the charge transfer complexes thereof, and/or the reduction products thereof.

According to the invention, a doped semiconductor layer is a combination of two substances or two compounds, in particular, of an semiconductor matrix material, and at least one doping agent. The doping agent serves as an electron acceptor.

Preferred is a doped semiconductor layer comprising a compound of the general formula (I)

Ce⁴⁺(L_{A}L_{B}L_{C}L_{D})⁴⁻ (I),

wherein
L_{A}; L_{B}; L_{C}; and L_{D} are independently from each other selected from a bidentate ligand having the general formula (II) 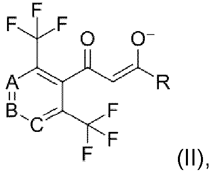
wherein A and C are independently selected from N (nitrogen), C (carbon)-H, C-D, C-Ar, C-OCF₃, C-SCF₃, SO₂CF₃, CN, F and CF₃;
wherein B is selected from N, C-D, C-Ar, C-H;
wherein R represents CF₃ or C₂F₅ or C₃F₇.

In an optional embodiment of the doped semiconductor layer L_{A}; L_{B}; L_{C}; and L_{D} are independently from each other selected from

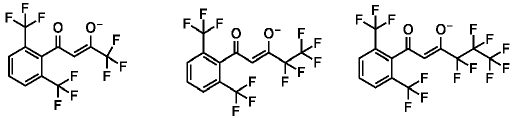

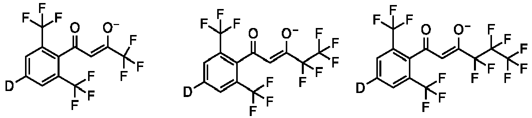

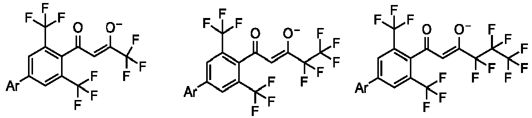

**Preferably** the **lateral conductivity** of the first doped semiconductor layer is smaller or than or equal to 2 E-5 S/cm, wherein the lateral conductivity is measured by a transmission line method at room temperature.

The semiconductor matrix material which may be called the matrix material can consist partially (> 10 or > 25% by weight) or substantially (> 50 % by weight or > 75% by weight) or totally (> 99 %) of a metal phthalocyanine complex, a porphyrine complex, especially metal porphyrine complex, oligothiophene-, oligophenyl-, oligophenylene vinylene- or oligofluorene compound, in which the oligomer preferably comprises 2-500 or more, preferably 2-100 or 2-50 or 2-10 or more monomeric units. The oligomer can also comprise > 4, > 6 or > 10 or more monomeric units, in particular also for the above-indicated ranges, thus, e.g., 4 or 6-10 monomeric units, 6 or 10-100 monomeric units or 10-500 monomeric units. Polymeric matrix materials can also be used. The monomers and oligomers can be substituted or unsubstituted and even block- or mixed polymerizates of the cited oligomers can be present as well as a compound with a triarylamine unit or a spiro-bifluorene compound.

The cited semiconductor matrix materials can also be present in combination with each other, optionally also in combination with other matrix materials. The semiconductor matrix materials can have electron-donating substitutents such as alkyl- or alkoxy groups that have a reduced ionizing energy or reduce the ionizing energy of the semiconductor matrix material.

The metal phthalocyanine complexes or porphyrine complexes used as semiconductor matrix material can have a main group metal atom or subgroup metal atom. The metal atom Me can be coordinated 4-, 5- or 6-fold, e.g., in the form of oxo(Me=O), dioxo-(O=Me=O) imine-, diimine-, hydroxo-, dihydroxo-, amino- or diamino complexes, without being limited to them. The phthalocyanine complex or porphyrine complex can each be partially hydrogenated, however, the mesomeric ring system is preferably not disturbed. The phthalocyanine can contain, e.g., magnesium, zinc, iron, nickel, cobalt, magnesium, copper or vanadyl (=VO) as central atom. The same or other metal atoms or oxometal atoms can be present in the case of porphyrine complexes.

In one preferred embodiment the semiconductor matrix material is either selected from phthalocyanine metal (Me) complexes (MePc) or triarylamino based or thiopheno based semiconductor matrix materials.

In particular, such dopable semiconductor matrix materials, in paritcular hole transport materials, can be arylated benzidines, e.g., N,N'-perarylated benzidines or other diamines such as of the type TPD (in which one, several or all of the aryl groups can have aromatic heteroatoms), suitable arylated starburst compounds such as N,N',N"-perarylated starburst compounds such as the compound TDATA (in which one, several or all of the aryl groups can have aromatic heteroatoms). The aryl groups can comprise phenyl, naphthyl, pyridine, quinoline, isoquinoline, peridazine, pyrimidine, pyrazine, pyrazole, imidazole, oxazole, furan, pyrrole, indole or the like, especially for each of the above-cited compounds. The phenyl groups of the particular compounds can be partially or completely replaced by thiophene groups.

It is understood that even other suitable semiconductor matrix materials, in particular hole-conducting materials can be used that have semi-conductive properties.

In particular a doped semiconductor layer comprises at least one semiconductor matrix material and at least one compound of the formula (I), wherein the semiconductor matrix material is selected from4,4',4"-tris(N-(2-naphthyl)-N-phenyl-amino)triphenylamine (2-TNATA), 4,4',4"-tris(N-3-methylphenyl-N-phenyl-amino)triphenylamine (m-MTDATA), N,N,N',N'-tetrakis(4-methoxy-phenyl)benzidine (MeO-TPD), (2,2',7,7'-tetrakis-(N,N-diphenylamino)-9,9'-spirobifluorene (spiro-TTB), N,N'-bis(naphthalen-1-yl)-N,N'-bis(phenyl)-benzidine, N,N'-bis(naphthalen-1-yl)-N,N'-bis(phenyl)-9,9-spiro-bifluorene, 9,9-bis[4-(N,N-bis-biphenyl-4-yl-amino)phenyl]-9H-fluorene, 2,2'-bis[N,N-bis(biphenyl-4-yl)amino]-9,9-spiro-bifluorene, N,N'-((9H-fluoren-9,9-diyl)bis(4,1-phenylen))bis(N-([1,1'-biphenyl]-4-yl)-[1,1'-biphenyl]-4-amine) (BPAPF), N,N'-bis(phenanthren-9-yl)-N,N'-bis(phenyl)-benzidine, 1,3,5-tris{4-[bis(9,9-dimethyl-fluoren-2-yl)amino]phenyl}benzene, tri(terphenyl-4-yl)amine, N-(4-(6-((9,9-dimethyl-9H-fluoren-2-yl)(6-methoxy-[1,1'-biphenyl]-3-yl)amino)-1,3,3-trimethyl-2,3-dihydro-1H-inden-1-yl)phenyl)-N-(6-methoxy-[1,1'-biphenyl]-3-yl)-9,9-dimethyl-9H-fluoren-2-amine, N-([1,1'-biphenyl]-4-yl)-N-(4-(6-([1,1'-biphenyl]-4-yl(9,9-dimethyl-9H-fluoren-2-yl)amino)-1,3,3-trimethyl-2,3-dihydro-1H-inden-1-yl)phenyl)-9,9-dimethyl-9H-fluoren-2-amine, N,N-di([1,1'-biphenyl]-4-yl)-3-(4-(di([1,1'-biphenyl]-4-yl)amino)phenyl)-1,1,3-trimethyl-2,3-dihydro-1H-inden-5-amine, N-(4-(6-(bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amine, N-(4-(6-(9,9'-spirobi[fluoren]-2-yl(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9'-spirobi[fluoren]-2-amine, N-(4-(6-(dibenzo[b,d]furan-2-yl(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)dibenzo[b,d]furan-2-amine, 9-(4-(6-(9H-carbazol-9-yl)-1,3,3-trimethyl-2,3-dihydro-1H-inden-1-yl)phenyl)-9H-carbazole, N-([1,1biphenyl]-4-yl)-3-(4-([1,1'-biphenyl]-4-yl(4-methoxyphenyl)amino)phenyl)-N-(4-methoxyphenyl)-1,1,3-trimethyl-2,3-dihydro-1H-inden-5-amine, 3-(4-(bis(6-methoxy-[1,1'-biphenyl]-3-yl)amino)phenyl)-N,N-bis(6-methoxy-[1,1'-biphenyl]-3-yl)-1,1,3-trimethyl-2,3-dihydro-1H-inden-5-amine, N1-([1,1'-biphenyl]-4-yl)-N1-(4-(6-([1,1'-biphenyl]-4-yl(4-(diphenylamino)phenyl)amino)-1,3,3-trimethyl-2,3-dihydro-1H-inden-1-yl)phenyl)-N4,N4-diphenylbenzene-1,4-diamine, N,N-di([1,1'-biphenyl]-4-yl)-4'-(6-(4-(di([1,1'-biphenyl]-4-yl)amino)phenyl)-1,3,3-trimethyl-2,3-dihydro-1H-inden-1-yl)-[1,1'-biphenyl]-4-amine, N-(4-(5-(bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amine, N-(4-(6-(bis(9,9-dimethyl-9H-fluoren-2-yl)amino)-1,3,3-trimethyl-2,3-dihydro-1H-inden-1-yl)-phenyl)-N-(9,9-dimethyl-9H-fluoren-2-yl)-9,9-dimethyl-9H-fluoren-2-amine, N,N'-bis(9,9-dimethyl-fluoren-2-yl)-N,N'-diphenyl-benzidine (BF-DPB), N,N'-((9H-fluorene-9,9-diyl)bis(4,1-phenylene))bis(N-([1,1'-biphenyl]-4-yl)-[1,1'-biphenyl]-4-amine) (BPAPF), N4,N4,N4',N4'-tetrakis(9,9-dimethyl-9H-fluoren-2-yl)-[1,1'-biphenyl]-4,4'-diamine (TDMFB), N-([1,1'-biphenyl]-2-yl)-N-(9,9-dimethyl-9H-fluoren-2-yl)-9,9'-spirobi[fluoren]-2-amine, (2,7-bis[N,N-bis(4-methoxyphenyl)amino]-9,9-spirobi[9*H*-fluorene] (spiro-MeO-TPD), a mixture of N-(4-(5-(bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amine and N-(4-(6-(bis(9,9-dimethyl-9H-fluoren-2-yl)amino)-1,3,3-trimethyl-2,3-dihydro-1H-inden-1-yl)phenyl)-N-(9,9-dimethyl-9H-fluoren-2-yl)-9,9-dimethyl-9H-fluoren-2-amine, N-([1,1'-biphenyl]-4-yl)-9,9-dimethyl-N-(4-(9-phenyl-9H-carbazol-3-yl)phenyl)-9H-fluoren-2-amine and mixtures thereof.

### Doping

In particular, the doping can be carried out such that the molar ratio of matrix molecules to the compound of the general formula (I), is in the range from 10000 : 1 to 1 : 1, preferably from 100 : 2 to 2 : 1, particularly preferably from 100 : 4 to 5 : 1.

Preferably, the compounds according to the invention are characterized by a preferential doping efficiency.

Advantageously, the compounds according to the invention lead to a conductivity of the doped semiconductor layer in the range of 1E-6 to 3E-5 S/cm, preferably between 2E-6 to 2E-5 S/cm. Thus, pixel crosstalk can be reduced, while the driving voltage of the OLED is advantageously low.

### Preparation of the doped semiconductor matrix layer

A further object of the invention relates to the doping of the respective semiconductor matrix material (hereinafter also referred to as hole transport material (HTM) which can also be referred to as hole conductor matrix) with the compounds of the general formula (I) according to the invention and used in accordance with the invention may be carried out by one or a combination of two or more than two of the following processes:
(a) Simultaneous evaporation of the hole transport material and the compound of the general formula (I), preferably in vacuum, preferably with one source for the HTM and one for at least one compound of general formula (I).
(b) Evaporation of a pre-mixed material containing the HTM and at least one compound of general formula (I) from one single source.
(c) Sequential deposition of the HTM and at least one compound of general formula (I). This process step is followed by diffusion of the dopant, i.e. the compound of the general formula (I) inward upon thermal treatment. The compound of general formula (I) might be deposited before or after HTM.
(d) Doping of a layer of HTM by a solution of at least one compound of general formula (I) followed by evaporation of the solvent by thermal treatment.
(e) Surface doping of a layer of a HTM by a layer of at least one compound of general formula (I) applied to one or both surfaces of the HT layer (hole transport layer), i.e. a layer containing an HTM.
(f) Preparing a solution of a HTM and at least one compound of general formula (I) and forming a film from the solution, e.g., by coating, casting, printing, or other film-forming techniques known to one skilled in the art.

The invention will be illustrated further with reference to the examples that follow, without restricting the scope to the specific embodiments described. The invention includes all combinations of described and especially of preferred features that do not exclude each other.

### EXAM PLES

### Abbreviation:

- TBME: tert.-Butylmethylether
- MTBE: Methyl-tert-butylether
- DCM: Dichlormethane
- DME: 1,2-Dimethoxyethane
- Spiro-TTB: 2,2',7,7'-tetrakis-(N,N-diphenylamino)-9,9'-spirobifluorene
- MeO-TPD: N,N,N',N'-tetrakis(4-methoxy-phenyl)benzidine
- Spiro-TAD: 2,2',7,7'-Tetrakis(N,N-diphenylamino)-2,7-diamino-9,9-spirobifluorene
- BPAPF: N,N'-((9H-fluorene-9,9-diyl)bis(4,1-phenylene))bis(N-([1,1'-biphenyl]-4-yl)-[1, 1'-biphenyl]-4-amine
- BCFA: N-([1,1'-biphenyl]-4-yl)-9,9-dimethyl-N-(4-(9-phenyl-9H-carbazol-3-yl)phenyl)-9H-fluoren-2-amine
- TCTA: 4,4',"-Tris(carbazol-9-yl)triphenylamine
- Al: Aluminium
- BPhen: 4,7-Diphenyl-1,10-phenanthrolin
- Cs: Caesium
- BAlq₂: Bis-(8-hydroxy-2-methylchinolin)-(4-phenylphenoxy)-aluminium
- TPBI: 2,2',2"-(1,3,5-Benzinetriyl)-tris(1-phenyl-1-H-benzimidazole)
- ppy: polypyrrole
- acac: acetylacetone
- TAPC: 1,1-Bis[(di-4-tolylamino)phenyl]cyclohexane
- ITO: Indium Tin Oxide
- EQE: External Quantum Efficiency
- PE: Power eFFICIENCY
- n.d.: not defines/measured

### EXAM PLES

The following examples illustrate the invention without limiting it in any way.

### Synthesis

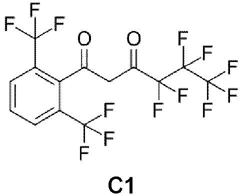

Ethyl heptafluorobutyrate (7.36 g, 30 mmol), NaOMe (1.2 g, 21.2 mmol) and 2,6-bis(trifluoromethyl)acetophenone (3.12 g, 12.2 mmol) were mixed in TBME (15 ml) at 60°C for 17 hours. The reaction mixture was diluted with ethyl acetate (20 ml) and 1M HCl (35 ml) was added. The organic phase was separated and washed with water and saturated NaCl. The organic phase was dried with MgSO₄, filtered, and the volatiles removed in vacuo yielding C1 as a pale yellow liquid (3.38 g, 64%). APCI-MS 452 [M-H]⁻.

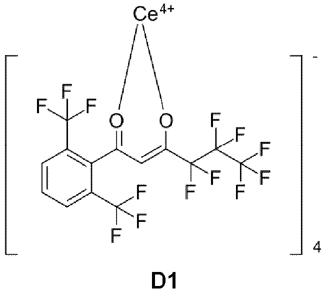

Diketone C1 (3.12 g, 6.9 mmol) was dissolved in TBME (50 ml) and cooled to 0°C and NaH (0.16 g, 6.9 mmol) was added. After the gas evolution ceased the volatiles were removed in vacuo. The residue was dissolved in acetonitrile (20 ml) and cerium ammonium nitrate (0.95 g, 1.7 mmol) was added. After 60 minutes, the suspension was filtered. The filter cake was rinsed with water (40ml) and dried in vacuo yielding the title product D1 as red crystalline solid (1.06 g, 32%). APCI-MS: 1944 [M]⁺.

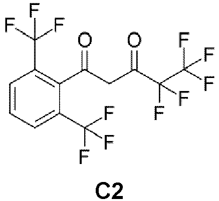

Ethyl pentafluoropropionate (5.45 g, 28.4 mmol), NaOMe (1.18 g, 21.3 mmol) and 2,6-bis(trifluoromethyl)acetophenone (3.64 g, 14.2 mmol) were mixed in TBME (20 ml) at 70°C for 12 hours. The reaction mixture was diluted with 1M HCl (25 ml) and TBME (15 ml). The organic phase was separated and washed with water and saturated NaCl. The organic phase was dried with MgSO₄, filtered, and the volatiles removed in vacuo. Hexane (20 ml) was added to the residue and sonicated. The suspension was filtered and dried in vacuo yielding the title compound C2 as colorless crystals (4.50 g, 79%). APCI-MS 401 [M-H]⁻.

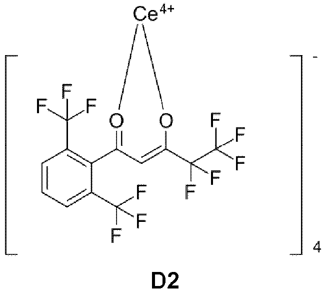

Diketone C2 (4.5 g, 11.2 mmol) was dissolved in TBME (40 ml) and cooled to 0°C and NaH (0.45 g, 11.2 mmol) was added. After the gas evolution ceased the volatiles were removed in vacuo. The residue was dissolved in acetonitrile (50 ml) and cerium ammonium nitrate (1.53 g, 2.8 mmol) was added. After 30 minutes, the volatiles were removed in vacuo. The mix was extracted with TBME (40 ml), filtered and rotated off in vacuo. DCM (20ml) was added to the residue and the mix was sonicated for 5 min and subsequently filtered to give the title product **D2** as red crystalline solid (1.29 g, 24%). APCI-MS: 1744 [M]⁺.

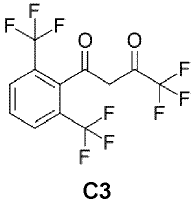

Ethyl trifluoroacetate (4.77 g, 33.8 mmol), NaOMe (1.2 g, 21.2 mmol) and 2,6-bis(trifluoromethyl)acetophenone (4.31 g, 16.8 mmol) were mixed in TBME (15 ml) at 65°C for 17 hours followed by 12 hours at 70°C. The reaction mixture was diluted with TBME (20 ml) and 1M HCl (35 ml) was added. The organic phase was separated and washed with NH₄Cl saturated solution followed and brine. The organic phase was dried with Na₂SO₄, filtered, and the volatiles removed in vacuo yielding an off-white solid that was sonicated for 10 min and stirred in petroleum ether for 12h and then filtered yielding the title compound **C3** as colorless crystals. (1.35 g, 23%).

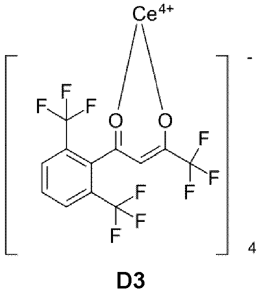

Diketone **C3** (4.04 g, 11.47 mmol) was dissolved in TBME (50 ml) and NaH (0.46 g, 11.5 mmol) was added. After the gas evolution ceased the volatiles were removed in vacuo. The residue was dissolved in acetonitrile (40 ml) and cerium ammonium nitrate (1.57 g, 2.87 mmol) was added. After 60 minutes, the suspension was filtered. The filter cake was rinsed with hexane (40 ml), water (40ml) and dried in vacuo yielding the title product **D3** as red crystalline solid. The solid was recrystallized from hot acetonitrile (1.3 g, 30%). APCI-MS: 1744 [M]⁻.

### Sample preparation and measurements

To determine the **evaporation temperature,** 15 mg of the material was filled in an aluminum oxide crucible of a point source and heated in a vacuum chamber (base pressure <5 × 10-7 mbar) at 5 K/min until an evaporation rate of 0.3 Å/s is reached. The evaporation rate is monitored with a quartz crystal microbalance (QCM). The rate is kept constant for 10 min by controlling the heating and the evaporation temperature is determined by measuring the temperature at the bottom of the crucible with a thermocouple. To achieve stable evaporation rates and to avoid cross contamination in mass production of organic electronic devices, such as OLEDs, it is advantageous to use materials with a high evaporation temperature of at least above 120°C. Advantageously, evaporation temperatures above 120°C reduce the risk of cross contamination during thermal evaporation in vacuum and are accordingly suitable for mass production.

For the measurement of the **lateral conductivity,** a transmission line method was used. Interdigitated ITO (indium tin oxide) electrodes were formed by photolithography on a glass substrate with four different channel lengths (40 µm, 80 µm, 120 µm, 160 µm). Then organic layers were deposited by thermal evaporation under ultrahigh vacuum conditions (base pressure <5 × 10-7 mbar) by controlling the evaporation rates with quartz crystal microbalances (QCMs). First, a 10 nm thick p-doped layer was prepared by co-evaporation of the p-dopant and the semiconductor matrix material, e.g. a hole transport material. To obtain a specific dopant concentration, the individual deposition rates were controlled with independent QCMs. These films were covered with 100 nm of intrinsic semiconductor matrix material to mimic the typical layer stack (common hole-injection layer + common hole-transport layer) in OLED display. Samples were encapsulated with glass lid and getter material under nitrogen atmosphere and characterized ex-situ at room temperature. Current-voltage characteristics (-10 V to 10 V) were measured using a source-measure unit Keithley 2400 and the lateral conductivity was determined from the slope of the resistance vs. channel length plot and by normalizing to the thickness of the p-doped layer, accounting for almost the entire current.

As semiconductor matrix material, HT-1 (N-([1,1'-biphenyl]-4-yl)-9,9-dimethyl-N-(4-(9-phenyl-9H-carbazol-3-yl)phenyl)-9H-fluoren-2-amine) and HT-2 (N-(9,9-Diphenyl-9H-fluoren-2-yl)-N,9-diphenyl-9H-carbazol-3-amine) were used. HT-1 and HT-2 have a HOMO level of about -5.15 eV and -5.08 eV, respectively, wherein the HOMO level is measured by cyclic voltammetry as described herein. Hole transport materials with a shallow HOMO > -5.2 eV, when determined by cyclic voltammetry as described herein, are typically used in OLED displays to ensure a good energy level alignment with the anode.

The **HOMO level** was determined by cyclic voltammetry measured with a platinum working and counter electrode, an Ag/AgCI reference electrode and Ferrocene/Ferrocenium as an internal standard and a scan rate of 0.1V/s. As electrolyte, acetonitrile with 0.1M tetrabutylammonium hexafluorophosphate was used. The HOMO level was calculated from E(HOMO) = -4.79 eV - E_{1/2}(oxidation vs. Fc/Fc+).

### Technical effect

### Evaporation temperature

**Table 1**

| p-Dopant | T_evap |
|---|---|
| CE-1 | 108°C |
| D1 | 131°C |
| D2 | 142°C |
| D3 | 132°C |

A technical effect of higher evaporation temperature is shown in Table 1. As can be seen from Table 1, comparative example CE-1 (

Tetrakis[1-[3,5-bis(trifluoromethyl)phenyl]-4,4,4-trifluoro-1,3-butanedionato-κO1,κO3]cerium, CAS 2624123-68-6) shows a low evaporation temperature of only 108°C. CE-1 is a material from the state of the art. , Low evaporation temperatures below 120°C entail a high risk of cross-contamination in production of multi-layer electronic devices, such as OLEDs. This applies in particular, because the onset of evaporation occurs at even lower temperatures. Examples D1, D2, and D3 show significantly higher evaporation temperatures of significantly above 120°C, even above 130°C. Accordingly, these materials can advantageously be used for mass production using thermal evaporation. To achieve stable evaporation rates and to avoid cross contamination in mass production of organic electronic devices, such as OLEDs, it is advantageous to use materials with a high evaporation temperature of at least above 120°C, preferably of above 125°C, more preferably of above 130°C. Advantageously, evaporation temperatures above 120°C reduce the risk of cross contamination during thermal evaporation in vacuum and are accordingly suitable for mass production.

### Conductivity

To optimize the electrical performance of an organic electronic component, particularly the voltage, it is beneficial that the conductivity of the doped semiconductor matrix material exceeds a certain value depending on the device structure and the application. For OLEDs, the hole injection layer generally requires a conductivity above 1 E-06 S/cm, preferably above 2E-6 S/cm, to enable a low operating voltage. On the other hand, as a disadvantage, a higher conductivity of a common hole-injection layer or a common hole transport layer leads to more severe **unintended pixel crosstalk.** Therefore, the doped layer should be optimized to a conductivity in such an advantageous range, preferably between 1E-6 S/cm and 3E-5 S/cm, more preferably between 2E-6 S/cm and 2E-5 S/cm. There are strong limitations to adjust the conductivity by reducing the doping concentration, because the control of the process is more challenging for very low doping concentrations. To reach a sufficient robustness and stability of the process a doping concentration of at least 2 vol.%, preferably 4 vol.% is required. Higher doping concentrations of at least 4 vol.% ensure a precise control of evaporation rate and accordingly a well-controlled distribution of dopant. Accordingly, for mass production an evaporation rate of at least 2 vol.%, preferably at least 4 vol.%, is desirable.

**Table 2**

| p-Dopant | HTM | Dopant concentration (vol%) | Lateral conductivity in S/cm |
|---|---|---|---|
| CE-1 | HT-1 | 2 | 3.2E-5 |
| CE-1 | HT-1 | 4 | 4.9E-5 |
| D1 | HT-1 | 2 | 0.5E-5 |
| D1 | HT-1 | 4 | 2.0E-5 |
| D2 | HT-1 | 2 | 0.5E-5 |
| D2 | HT-1 | 4 | 1.3E-5 |
| D3 | HT-1 | 3 | 0.2E-5 |
| CE-1 | HT-2 | 2 | 0.7E-5 |
| CE-1 | HT-2 | 4 | 1.0E-5 |
| D1 | HT-2 | 2 | 0.4E-5 |
| D1 | HT-2 | 4 | 0.7E-5 |
| D2 | HT-2 | 2 | 0.2E-5 |
| D2 | HT-2 | 4 | 0.3E-5 |
| D3 | HT-2 | 6 | 0.2E-5 |

The technical effect of optimized conductivity is shown in Table 2, which shows that examples D1, D2, and D3 lead to conductivities above 1E-6 S/cm and should therefore be suitable for the use in OLEDs. At the same time the conductivities obtained with the examples D1, D2, and D3 are lower than the ones obtained with comparative example 1 at the same dopant concentration. Even at a low dopant concentration of 2 vol%, comparative example 1 cannot reach the same low conductivities as examples 1 and 2. Accordingly, examples D1, D2, and D3 are advantageous for the production of OLED displays with a low lateral conductivity of the common hole injection and hole transport layer and thus reduced pixel crosstalk.

To verify that D1 and D2 are suitable for OLED production, an OLED structure was prepared as follows. For Example 1, a glass substrate with pre-patterned 130 nm ITO anode was ultrasonically washed with dimethyl sulfoxide for 20 minutes, rinsed by Di-water for 5 minutes and ultrasonically washed with first Di-water and then with ethanol for 10 minutes. The substrate was then rinsed with Di-water and consequently dry-spined at 1500 rpm in a nitrogen stream, followed by oxygen plasma treatment to prepare the anode surface.

Then, 10 nm of HT-1 (9H-Fluoren-2-amine, N-[1,1'-biphenyl]-4-yl-9,9-dimethyl-N-[4-(9-phenyl-9H-carbazol-3-yl)phenyl]) doped with 2 vol% of D1 was vacuum deposited to form a hole-injection layer (HIL). Then, HT-1 was vacuum deposited on top of HIL to form hole transport layer (HTL) with a thickness of 100 nm. Then, an electron-blocking layer (EBL) was formed on the HTL by depositing 4-[Bis(1,1'-biphenyl-4-yl)amino]-4'-(9H-carbazol-9-yl)-1,1'-biphenyl with a thickness of 10 nm.

Then, a blue-emitting emission layer (EML) consisting of 97 vol% 2-Methyl-9,10-di-2-naphthalenylanthracene (MADN)as EML host and 3 vol% 4,4'-([1,1'-Biphenyl]-4,4'-diyldi-2,1-ethenediyl)bis[N,N-bis(4-methylphenyl)benzenamine (DPAVBi ) as fluorescent blue emitter dopant was vacuum deposited on the EBL with a thickness of 20 nm.

Then, a hole-blocking layer (HBL) was formed on the EML with a thickness of 10 nm by depositing 4,7-Diphenyl-1,10-phenanthroline (BPhen). The electron transport layer (ETL) was vacuum deposited on the HBL by co-evaporation of 4,7-Diphenyl-1,10-phenanthroline (BPhen) and Cs with a ratio of 1:1 and a thickness of 25 nm. Then, 100 nm of Al was deposited on the ETL to form a cathode layer.

The OLED structure was protected from ambient conditions by encapsulation glass lid that forms a cavity. This cavity contains a getter material, further ensuring the integrity and longevity of the OLED structure.

The OLED structure of Example 2 was prepared the same way as Example 1 but with 4 vol% of D1 instead of 2 vol%.

The OLED structure of Example 3 was prepared the same way as Example 2 but with 4 vol% of D2 instead of D1.

The OLED structure of Comparative Example 1 was prepared in the same way as Example 1, but with 2 vol% of CE-1 instead of 2 vol% of D1.

The electro-optical performance parameters of the OLEDs were measured at the room temperature. The current-voltage characteristic is measured using a source-measure unit Keithley 2400. The voltage is applied with the step of 0.1 V in the range from -2 V to 6 V. The OLEDs are further measured in the integrating sphere to calculate absolute external quantum efficiency. The integrating sphere is covered by a highly reflective white material so that all the emitted light from the OLED's hemisphere is collected by a detector. The OLEDs are measured in the same way as explained above. The intensity is measured with the spectrometer OceanOptics USB 4000 and the photodiode. The lifetime measurements are performed at the constant current density of 10 mA/cm2. The change in luminance and voltage are recorded every 600 s. The LT90 values are reported after 10% luminance drop.

**Table 3**

| | p- Dopant | Dopant concentration (vol%) | Voltage [V] at 10 mA/cm² | External quantum efficiency [%] at 10 mA/ cm² | LT70 [h] at 10 mA/ cm² | V(LT70)/V0 |
|---|---|---|---|---|---|---|
| Comparative Example 1 | CE-1 | 2 | 3.6 | 7.3 | 22 | 1.13 |
| Example 1 | D1 | 2 | 3.6 | 7.4 | 26 | 1.14 |
| Example 2 | D1 | 4 | 3.5 | 7.4 | 30 | 1.13 |
| Example 3 | D2 | 4 | 3.5 | 7.3 | 28 | 1.14 |

Table 3 shows the technical effect. Example 1 to 3 have the same or even lower driving voltage and the same or even higher external quantum efficiency compared to Comparative Example 1. The lifetime of Example 1 to 3 is improved compared to Comparative Example 1. This means it is advantageous to use compounds D1 and D2 as p-dopants at a concentration of 2 or 4 vol% as compared to CE-1 at 2 vol%. As can be seen in Table 2, the lateral conductivity with D1 and D2 in HT-1 at both 2 and 4 vol% doping ratio is smaller than with 2 vol% of comparative example 1. Therefore, D1 and D2 can advantageously be used in OLED to obtain good electro-optical performance and low lateral conductivity.

In summary, the examples show both high evaporation temperatures suitable for mass production and suitable conductivities that allow for application in OLED display and reduction of pixel crosstalk.

## Claims

1. An OLED **display** comprising a plurality of OLED pixels, each of the OLED pixels comprising
- An anode,
- a cathode,
- at least a first **emission layer,** and
wherein the cathode of at least a first OLED pixel and a second OLED pixel forms a **common cathode,**
wherein at least the first OLED pixel and the second OLED pixel comprise a first p-type semiconductor layer that forms at least a first **common p-type semiconductor layer,**
wherein the first common p-type semiconductor layer comprises a compound of the general formula (I)
Ce⁴⁺(L_{A}L_{B}L_{C}L_{D})⁴⁻ (I),
wherein
L_{A}; L_{B}; L_{C}; and L_{D} are independently from each other selected from a bidentate ligand having the general formula (II) 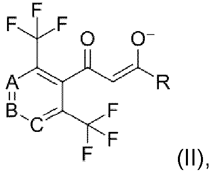
wherein A and C are independently selected from N (nitrogen), C (carbon)-H, C-D, CAr, C-OCF₃, C-SCF₃, SO₂CF₃, CN, F and CF₃;
wherein B is selected from N, C-D, C-Ar, C-H;
wherein R represents CF₃ or C₂F₅ or C₃F₇.

2. The OLED display according to claim 1, wherein L_{A}; L_{B}; L_{C}; and L_{D} are similar.

3. The OLED display according to claim 1 or 2, wherein A and C are independently selected from N (nitrogen), C (carbon)-H, C-D, C-Ar, C-OCF₃, C-SCF₃, SO₂CF₃, CN, and F.

4. The OLED display according to one of the claims 1 to 3, wherein L_{A}; L_{B}; L_{C}; and L_{D} are independently from each other selected from

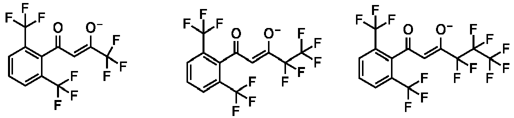

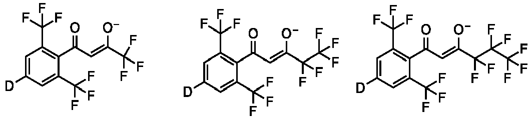

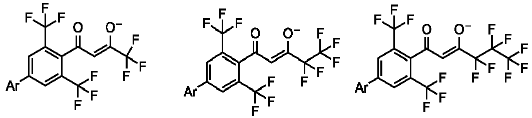

5. An OLED display according to one of the claims 1 to 4, wherein the first common p-type semiconductor layer is positioned between the anode and the first emission layer.

6. The OLED display according to one of the claims 1 to 5, wherein the first common p-type semiconductor layer is a hole transport layer and/or a hole injection layer and/or a charge generation layer.

7. The OLED display according to one of the claims 1 to 6, wherein at least 100 OLED pixels of the plurality of pixels comprise a cathode that forms a common cathode and wherein at least 100 OLED pixels of the plurality of pixels comprise a first p-type semiconductor layer that form the first common p-type semiconductor layer.

8. The OLED display according to one of the claims 1 to 7, wherein the **lateral conductivity** of the first common p-type semiconductor layer is equal to or smaller than 2 E-5 S/cm, wherein the lateral conductivity is measured by a transmission line method at room temperature.

9. The OLED display according to one of the claims 1 to 8, wherein the first common p-type semiconductor layer comprises a semiconductor matrix material.

10. The OLED display according to claim 9, wherein the HOMO level of the semiconductor matrix material, is equal to or higher than the HOMO of BPAPF if measured with the same method.

11. The OLED display according to one of the claims 1 to 10, wherein the first OLED pixel and the second OLED pixel comprise at least a second emission layer and the first common p-type semiconductor layer is a first common charge generation layer, wherein the first common **charge generation layer** is arranged between the first emission layer and the second emission layer.

12. The OLED display according to one of the claims 1 to 11, wherein the OLED display comprises a second common p-type semiconductor layer which comprises a compound of formula (I) according to one of the claims 1 to 10.

13. The OLED display according to claim 12, wherein the second common p-type semiconductor layer is a p-type charge generation layer or a hole injection layer or a hole transport layer.

14. A doped semiconductor matrix material comprising at least one electron donor and at least one compound of the formula (I)
Ce⁴⁺(L_{A}L_{B}L_{C}L_{D})⁴⁻ (I),
wherein
L_{A}; L_{B}; L_{C}; and L_{D} are independently from each other selected from a bidentate ligand having the general formula (II) 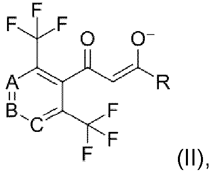
wherein A and C are independently selected from N (nitrogen), C (carbon)-H, C-D, CAr, C-OCF₃, C-SCF₃, SO₂CF₃, CN, F and CF₃;
wherein B is selected from N, C-D, C-Ar, C-H;
wherein R represents CF₃ or C₂F₅ or C₃F₇.

15. A method to produce an OLED display according to one of the claims 1 to 13, comprising at least one of the following steps:
(a) Simultaneous evaporation of the semiconductor matrix material and the compound of formula (I),
(b) Evaporation of a pre-mixed material containing the semiconductor matrix material and at least one compound of general formula (I) from one single source,
(c) Sequential deposition of the semiconductor matrix material and at least one compound of general formula (I),
(d) Doping of a semiconductor matrix material layer by a solution of at least one compound of general formula (I) followed by evaporation of the solvent by thermal treatment,
(e) Surface doping of a layer of a semiconductor matrix material by a layer of at least one compound of general formula (I) applied to one or both surfaces of the layer of a semiconductor matrix material,
(f) Preparing a solution of a semiconductor matrix material and at least one compound of general formula (I) and forming a film from the solution.
